(19)

Europäisches Patentamt
European Patent Office
Office européen des brevets

(11)     **EP 2 570 470 A1**

(12)     **EUROPEAN PATENT APPLICATION**

(43) Date of publication:
**20.03.2013  Bulletin 2013/12**

(51) Int Cl.:
*C10G 45/40* (2006.01)          *B01J 23/50* (2006.01)
*C07C 5/08* (2006.01)          *C07C 7/167* (2006.01)

(21) Application number: **11181793.8**

(22) Date of filing: **19.09.2011**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Shell Internationale Research
Maatschappij B.V.
2596 HR The Hague (NL)**

(72) Inventor: **The designation of the inventor has not
yet been filed**

(74) Representative: **Matthezing, Robert Maarten et al
Shell International B.V.
Intellectual Property Services
P.O. Box 384
2501 CJ The Hague (NL)**

(54)     **Hydrogenation catalyst**

(57)     The present invention relates to a Pd-catalyst, comprising palladium and at least two metals selected from the group consisting of platinum, silver, manganese and bismuth, to its preparation and to its use as a selective hydrogenation catalyst for unsaturated hydrocarbons, particularly acetylene.

EP 2 570 470 A1

**Description**

The present invention relates to palladium catalysts, a method for their production and their use in a hydrogenation process.

In refineries and petrochemical facilities large amounts of hydrocarbons are produced and stored which comprise significant amounts of unsaturated hydrocarbons causing problems during the further processing steps and its storage. Such unsaturated compounds are for instance acetylene, propyne, propadiene, butadienes, vinylacetylene, butynes, phenylacetylene and styrene.

Acetylene is known to reduce the catalyst activity in polymerisation processes and the quality of the polymers is deteriorated. Thus, in the synthesis of polyethylene from ethylene the concentration of acetylene should be minimized.

The undesired, unsaturated compounds are removed mainly by selective hydrogenation wherein these compounds are hydrogenated, preferably to a content of less than a few ppm. It is important for the efficiency of the selective acetylene hydrogenation that the hydrogenation of ethylene to ethane and the oligomerization to higher hydrocarbons and the production of coke are avoided.

In the art, it was known to use supported Pd-catalysts based on aluminium oxides and silicium oxides for selective hydrogenation processes.

To improve the selectivity of the catalyst in hydrogenation processes FR2603578 discloses a $Pd/Al_2O_3$ catalyst with gold as promoting metal. US2802889 discloses a method for the selective hydrogenation of acetylene with Pd-catalysts comprising copper, silver, and gold wherein the content of the elements of groups 8-10 in the metallic phases is 1 to 40 %. DE19757990 discloses supported Pd catalysts comprising Ni, Co, or Ag and Na, K, or Ca, and a layer of precipitated organic silanes.

US4484015 discloses a Pd-Ag/$\alpha$Al$_2$O$_3$ catalyst wherein Ag is homogeneously distributed over the total catalyst and 90 % of the Pd is distributed in the outer shell region with a penetration depth of 300 $\mu$m. DE19840373 discloses catalysts comprising at least one metal of the eighth subgroup with an eggshell distribution and at least one metal of the first subgroup with a homogeneous distribution over the entire catalyst. KR20000059743 discloses the promotion of Pd with titanium oxide, whereby the selectivity of the Pd shall be increased by the SMSI effect. US7453017 discloses a method wherein the SMSI effect is induced at already 300° C. US6255548 discloses supported Pd catalysts promoted with Ge, Sn, Pb, Re, Ga, In, Au, Ag, and Tl. W02011107565 discloses a catalyst comprising palladium and at least one promoter selected from the group consisting of rare earth metals, hafnium and tungsten, and use thereof as a selective hydrogenation catalyst for unsaturated hydrocarbons, particularly acetylene.

According to the state of the art catalysts exhibit an unsatisfactory durability and selectivity, in particular when employed in a hydrogenation process, in particular in selective hydrogenation processes for hydrogenating acetylene, propyne, propadiene, butadienes, vinylacetylene, butynes, phenylacetylene and styrene in gaseous phase.

Therefore, the technical problem underlying the present invention is to overcome the above-identified disadvantages, in particular to provide a catalyst to be suitable for the selective hydrogenation of unsaturated hydrocarbons in a hydrocarbon feed, particularly in a gaseous hydrocarbon feed, comprising unsaturated hydrocarbons, in particular acetylene, propyne, propadiene, butadienes, vinylacetylene, butynes, phenylacetylene and styrene, which shows a high, in particular improved, durability, selectivity, activity or a combination of two or three thereof, a method for producing said catalyst and its use for the hydrogenation of a hydrocarbon feed.

Furthermore, the technical problem underlying the present invention is to provide a process for the hydrogenation of a hydrocarbon feed, in particular for the selective hydrogenation of unsaturated hydrocarbons in a hydrocarbon feed, particularly in a gaseous hydrocarbon feed, comprising unsaturated hydrocarbons, in particular acetylene, propyne, propadiene, butadienes, vinylacetylene, butynes, phenylacetylene and styrene, which overcomes the above identified disadvantages and in particular which is characterized by a high, in particular improved activity, durability, selectivity or a combination of two or three thereof.

The technical problem of the present invention is solved according to the teaching of the independent claims.

In particular, the present invention solves its technical problem by the provision of a Pd-catalyst, comprising palladium and at least two metals selected from the group consisting of platinum, silver, manganese and bismuth. Accordingly, the present invention relates to such Pd-catalyst.

In the context of the present invention the term "promoter" means one, more or all of the above-identified elements.

Preferably, said at least two metals in the Pd-catalyst according to the present invention comprise a first metal which is platinum or silver and a second metal which is manganese or bismuth. Thus, in the context of the present invention a "first metal" is platinum or silver and a "second metal" is manganese or bismuth

In the context of the present invention a "liquid medium" is preferably a solution. In the context of the present invention a "liquid medium" may also be a suspension. In a preferred embodiment of the present invention the solution or suspension is an aqueous solution or suspension.

In the context of the present invention the expression "and/or" used in between two elements is meant to designate that both elements linked by said term are referred to in a cumulative or alternative manner. Thus, the expression "A and/or B" encompasses the meanings "A or B" and "A and B", that means "any one of A, B or both".

"% by weight" or "wt.%" values given in the present teaching refer, if not otherwise stated, to the weight of the total dry catalyst. In the context of the present invention, the components of the catalyst are to be selected in an overall amount to add up to 100 % by weight, most preferably not to exceed 100 % by weight.

In the context of the present invention, the term "outer shell region" also called "egg shell region" is the peripheral, preferably outer peripheral, region of the catalyst between the surface of the catalyst and its centre having a maximum depth calculated along an axis in the diffusion direction of impregnating metal ions towards the catalyst interior, in particular along an axis drawn perpendicularly to the tangent of a given point of the surface of the catalyst towards the interior of the catalyst, of 400 $\mu$m, 350 $\mu$m, 300 $\mu$m, 250 $\mu$m, preferably 220 $\mu$m, preferably 200 $\mu$m and most preferably 150 $\mu$m and wherein said outer shell region is characterised by the presence of preferably at least 90% of the total amount of palladium. Thus, the depth of the outer shell region is the cross-sectional depth of the outer shell region and therefore equals the distance between the geometrical surface of the catalyst and the circumference of the catalyst centre.

In the content of the present invention the "outer shell region" is therefore defined by the presence of preferably at least 90% of the total amount of palladium within a maximal depth of 400 $\mu$m from the surface of the catalyst. Thus, the peripheral or outer shell region of the catalyst is the region, wherein preferably at least 90% of the total amount of palladium contained in the total catalyst is present and which extends from the surface of the catalyst to not more than 400 $\mu$m, 350 $\mu$m, 300 $\mu$m, 250 $\mu$m, preferably 220 $\mu$m, preferably 200 $\mu$m and most preferably 150 $\mu$m beneath the geometrical surface of the catalyst.

In the context of the present invention, the centre of the catalyst is the remainder of the catalyst, i. e. the interior region of the catalyst.

In a preferred embodiment of the present invention the diameter of the centre of the catalyst is at least 2, 3, 4, 5, 6, 7, 8, 9, 10, 11, 12, 13, 14 or 15 times greater than the depth of the outer shell region.

In a preferred embodiment of the present invention the radius of the centre of the catalyst is at least 2, 3, 4, 5, 6, 7, 8, 9, 10, 11, 12, 13, 14 or 15 times greater than the depth of the outer shell region.

In the context of the present invention, the term "support" or "carrier" is understood as being a pure carrier containing no catalytically active metal or as a carrier which comprises at least one catalytically active metal, preferably at least one metal selected from the group consisting of palladium, platinum, silver, manganese and bismuth. Said catalytically active metal being preferably already present in the catalyst support is distributed homogeneously or inhomogeneously over the entire catalyst support.

Further, the present invention relates to a method for preparing a Pd-catalyst comprising combining palladium with at least two metals selected from the group consisting of platinum, silver, manganese and bismuth. The method of the present invention uses, for instance, as a support either a carrier containing no catalytically active metal or in another embodiment a carrier which already contains at least one catalytically active metal, preferably at least one metal selected from the group consisting of palladium, platinum, silver, manganese and bismuth, and which is therefore in a subsequent process step further impregnated with at least one further metal, in particular at least one metal selected from the group consisting of palladium, platinum, silver, manganese and bismuth.

In the context of the present invention, the term "inhomogeneous" means that a metal is unevenly distributed in the entire catalyst. The inhomogeneous distribution of the metal is preferably obtained by impregnation of the support with the catalytically active metal.

Other methods to obtain an inhomogeneous distribution may be used and are known in the art. Accordingly, by an appropriate choice of the support material, the metals and/or metal precursors used and the process conditions, in particular the pH and the temperature, an inhomogeneous distribution may also be achieved, even if the liquid medium is homogeneously distributed in the entire catalyst. Thus, in one embodiment an inhomogeneous distribution of a metal may also be achieved by a homogeneous distribution of the liquid media used in the present invention. Further methods, such as precipitation methods, may be used to achieve the inhomogeneous distribution.

In another preferred embodiment a homogeneous distribution of the metal may be achieved by impregnation, precipitation or other methods by applying appropriate process conditions.

In the context of the present invention, the term "homogeneous" means that a metal is evenly distributed in the entire catalyst. The homogenous distribution of the metal is preferably obtained by mixing, extrusion, coextrusion and co-granulation with a support precursor as to obtain a support, for instance provided in below-mentioned method steps (a) and (i).

In the context of the present invention the term "homogeneous distribution of the liquid medium" means that all accessible surface areas and volumetric regions of at least one, preferably all, species, for instance micropores and macropores, of the pore system are filled, moistened or covered homogeneously.

In the context of the present invention the term "selective hydrogenation of a hydrocarbon feed" is meant to designate a process according to which a hydrocarbon feed containing two groups of unsaturated hydrocarbons is subjected to a hydrogenation, wherein a first group of specific unsaturated hydrocarbons, in particular highly unsaturated hydrocarbons such as alkynes and di- or oligounsaturated hydrocarbons such as alkadienes, alkatrienes or alkapolyenes, in particular acetylene, butadiene, propyne and propadiene, is hydrogenated and wherein a second group of other less unsaturated hydrocarbons, in particular monounsaturated hydrocarbons, namely alkenes such as ethylene, remains substantially in the unsaturated form. Thus, a selective hydrogenation according to the present invention is preferably a hydrogenation wherein a first group of specific unsaturated hydrocarbons, in particular comprising acetylene, is hydrogenated, preferably to ethylene, and a second group of other specific unsaturated hydrocarbons, in particular comprising ethylene, is not hydrogenated and remains monounsaturated.

In the context of the present invention the term "hydrocarbons" refers both to hydrocarbons consisting solely of carbon and hydrogen atoms and to hydrocarbons additionally comprising at least one functional group and/or at least one heteroatom, for instance nitroaromatics.

In a particularly preferred embodiment the term "hydrocarbons" refers to hydrocarbons consisting solely of carbon and hydrogen atoms.

In a furthermore preferred embodiment the term "hydrocarbons" refers to hydrocarbons additionally comprising at least one functional group and/or at least one heteroatom, for instance nitroaromatics.

Thus, the present invention foresees a Pd-catalyst, wherein palladium and at least two metals selected from the group consisting of platinum, silver, manganese and bismuth are distributed independently from each other homogeneously or inhomogeneously over the entire catalyst.

In a preferred embodiment of the present invention the Pd-catalyst is in the form of a catalyst mesh or catalyst gauze. In a preferred embodiment the Pd-catalyst being in the form of a catalyst mesh or catalyst gauze comprises palladium and the at least two metals selected from the group consisting of platinum, silver, manganese and bismuth.

In a preferred embodiment of the present invention the Pd-catalyst comprises a support. Therefore, it is foreseen that the Pd-catalyst is preferably a supported Pd catalyst. The preferred supported Pd-catalyst comprises palladium and at least two metals selected from the group consisting of platinum, silver, manganese and bismuth, and a support.

In a preferred embodiment the metals selected from the group consisting of palladium, platinum, silver, manganese and bismuth are distributed independently from each other homogeneously or inhomogeneously over the entire catalyst.

In a preferred embodiment of the present invention the Pd catalyst comprises a monolithic or honeycomb body. In a preferred embodiment of the present invention the monolithic or honeycomb-based catalyst comprises a monolithic or honeycomb body which can be an inert ceramic or metallic carrier, in particular traversed by parallel floating channels.

In a preferred embodiment of the present invention the metals selected from the group consisting of palladium, platinum, silver, manganese and bismuth are applied, preferably by impregnating, to the monolithic or honeycomb body.

In a preferred embodiment of the present invention the monolithic or honeycomb-based Pd-catalyst comprises a support, which support is present in form of a layer with a minimum thickness of 200 $\mu$m, preferably 150 $\mu$m and particularly 100 $\mu$m on parts of, preferably on the entire, monolithic or honeycomb body and wherein in said support layer Pd and the at least two metals selected from the group consisting of platinum, silver, manganese and bismuth are distributed independently from each other homogeneously or inhomogeneously over the entire support.

In a preferred embodiment of the present invention the outer shell region of the monolithic or honeycomb-based Pd catalyst has a maximum depth of 100 $\mu$m, preferably 50 $\mu$m, preferably 30 $\mu$m and in particular 20 $\mu$m, wherein 90% of the total amount of the at least one metal selected from the group consisting of palladium, platinum, silver, manganese and bismuth is distributed in the outer shell region.

Thus, the invention foresees in a preferred embodiment of the present invention that palladium and the at least two metals selected from the group consisting of platinum, silver, manganese and bismuth are distributed independently from each other homogeneously or inhomogeneously over the entire supported catalyst.

In a preferred embodiment of the present invention palladium and the at least two metals selected from the group consisting of platinum, silver, manganese and bismuth are distributed independently from each other homogeneously or inhomogeneously over the entire supported catalyst.

In the present invention, the Pd-catalyst comprises at least three metals. Preferably, the Pd-catalyst comprises three and no further metals. In addition to palladium, the Pd-catalyst preferably comprises a first metal which is platinum or silver and a second metal which is manganese or bismuth.

In a preferred embodiment of the present invention, said first metal is platinum and said second metal is bismuth. That is to say, preferably, the Pd-catalyst is a Pd-Pt-Bi-catalyst.

In another preferred embodiment of the present invention, said first metal is silver and said second metal is manganese. That is to say, preferably, the Pd-catalyst is a Pd-Ag-Mn-catalyst.

Further, it is envisaged in the present invention that said first metal is platinum and said second metal is manganese, that is to say a Pd-Pt-Mn catalyst, or that said first metal is silver and said second metal is bismuth, that is to say a Pd-Ag-Bi catalyst.

In a preferred embodiment of the invention the palladium is distributed homogeneously over the entire catalyst.

In a preferred embodiment of the present invention the Pd-catalyst comprises an outer shell region having a maximum penetration depth of 400 $\mu$m and a centre, wherein at least 90% of the total amount of palladium is distributed in the outer shell region.

In a preferred embodiment of the present invention the at least two metals selected from the group consisting of platinum, silver, manganese and bismuth are distributed homogeneously over the entire catalyst.

In a preferred embodiment of the present invention the Pd-catalyst comprises an outer shell region having a maximum penetration depth of 400 $\mu$m and a centre, wherein at least 90% of the total amount of the at least two metals selected from the group consisting of platinum, silver, manganese and bismuth are distributed in the outer shell region.

In a preferred embodiment of the present invention all metals selected from the group consisting of palladium, platinum, silver, manganese and bismuth are distributed inhomogeneously in the supported Pd-catalyst, preferably 90% of the total amount of the said metals is distributed in the outer shell region.

In a preferred embodiment of the present invention the weight ratio of palladium to the total of the first and second metals is 10:1 to 1:10, more preferably 2:1 to 1:4.

In a preferred embodiment of the present invention the weight ratio of palladium to the first metal is 10:1 to 1: 10, more preferably 3:1 to 1:3.

In a preferred embodiment of the present invention the weight ratio of palladium to the second metal is 10:1 to 1:10, more preferably 5:1 to 1:1.

In a preferred embodiment a supported Pd-catalyst is provided comprising palladium and at least two metals selected from the group consisting of platinum, silver, manganese and bismuth and a support, which catalyst comprises an outer shell region having a maximum depth of 400 $\mu$m, 350 $\mu$m, 300 $\mu$m, 250 $\mu$m, 220 $\mu$m, 200 $\mu$m or 150 $\mu$m and a centre, wherein at least 90% of the total amount of palladium and at least 90% of the total amount of the at least two metals selected from the group consisting of platinum, silver, manganese and bismuth are distributed in the outer shell region.

In a preferred embodiment, the present invention provides a catalyst, wherein the concentration maximum of the at least one metal selected from the group consisting of palladium and the at least two metals selected from the group consisting of platinum, silver, manganese and bismuth is located in the outer shell region, namely in a subregion thereof within a depth from 50 to 200 $\mu$m, preferably 50 to 150 $\mu$m, preferably 70 to 130 $\mu$m, preferably 90 to 110 $\mu$m, calculated from the surface of the catalyst towards its centre. Thus, the invention foresees in a preferred embodiment that within the outer shell region as defined above, that means within the peripheral region of the catalyst, wherein preferably at least 90% of the total amount of palladium and the at least two metals selected from the group consisting of platinum, silver, manganese and bismuth are located and which is defined by a maximal depth of 400 $\mu$m beneath the geometrical surface, that means counted from the surface of the catalyst, a subregion is present, in which a concentration maximum of the palladium and the at least two metals selected from the group consisting of platinum, silver, manganese and bismuth is present and wherein said subregion is located in a region from a depth from 50 $\mu$m counted from the surface of the catalyst to 200 $\mu$m counted from the surface of the catalyst. Thus, the maximum of the concentration of the palladium and the at least two metals selected from the group consisting of platinum, silver, manganese and bismuth is preferably contained in a layer in a specified region of the outer shell region.

In a preferred embodiment a catalyst of the present invention is a catalyst comprising palladium and at least two metals selected from the group consisting of platinum, silver, manganese and bismuth, that means is a three- or multi-metal catalyst, and a support, which catalyst comprises an outer shell region having a maximum depth of 400 $\mu$m, 350 $\mu$m, 300 $\mu$m, 250 $\mu$m, 220 $\mu$m, 200 $\mu$m or 150 $\mu$m and a centre, wherein at least 90% of the total amount of palladium and 90% of the at least two metals selected from the group consisting of platinum, silver, manganese and bismuth are distributed in the outer shell region. In a particularly preferred embodiment a catalyst according to the above is provided, wherein the concentration maximum of palladium and the at least two metals selected from the group consisting of platinum, silver, manganese and bismuth are located in the outer shell region in a subregion thereof which is located in a layer in a depth from 50 to 200 $\mu$m of the catalyst. The catalyst prepared by the present invention surprisingly shows a very high catalyst lifetime, e.g. durability, a high activity and a particular high selectivity for the unsaturated hydrocarbons to be removed.

In a preferred embodiment of the present invention at least 90 %, preferably at least 91 %, 92 %, 93 %, 94 %, 95 %, 96 %, 97 %, 98 %, 99 % and most preferably 100 % of the total amount of the at least two metals selected from the group consisting of platinum, silver, manganese and bismuth is distributed in the outer shell region.

In a preferred embodiment of the present invention at least 90 %, preferably at least 91 %, 92 %, 93 %, 94 %, 95 %, 96 %, 97 %, 98 %, 99 % and in particular 100 % of the total amount of the palladium is distributed in the outer shell region.

In a preferred embodiment of the present invention at least 90 %, preferably at least 91 %, 92 %, 93 %, 94 %, 95 %, 96 %, 97 %, 98 %, 99 % and in particular 100 % of the total amount of the platinum is distributed in the outer shell region.

In a preferred embodiment of the present invention at least 90 %, preferably at least 91 %, 92 %, 93 %, 94 %, 95 %, 96 %, 97 %, 98 %, 99 % and in particular 100 % of the total amount of the palladium and the at least two metals selected from the group consisting of platinum, silver, manganese and bismuth is distributed in the outer shell region.

In a further preferred embodiment of the present invention, the palladium and the at least two metals selected from the group consisting of platinum, silver, manganese and bismuth are the only metals, in particular the only catalytically active metals in the entire catalyst.

In a preferred embodiment of the present invention the palladium concentration and the concentration of each of the at least two metals selected from the group consisting of platinum, silver, manganese and bismuth are from 0.001 to 5.0 % by weight, more preferably 0.005 to 5.0 % by weight, more preferably 0.01 to 5.0 % by weight, more preferably 0.1 to 4.0 % by weight, more preferably 0.2 to 3.0 % by weight and in particular 0.2 to 1.6 % by weight (based on the total weight of the catalyst). Said concentrations may also be from 0.005 to 0.1 % by weight.

In a preferred embodiment of the present invention the support is selected from the group consisting of C, $TiO_2$, $Al_2O_3$, $ZrO_2$ and $SiO_2$. As catalyst support, also modifications of C, $TiO_2$, $Al_2O_3$, $ZrO_2$ and $SiO_2$ can be used. Preferably, combinations of the catalyst supports selected from the group consisting of C, $TiO_2$, $Al_2O_3$, $ZrO_2$ and $SiO_2$ can be used.

In a preferred embodiment of the present invention the catalyst support is $Al_2O_3$ or a modification thereof.

In a preferred embodiment of the present invention the catalyst may be in the form of hollow cylinders, tablets, spheres or extrudates.

In a preferred embodiment of the present invention the catalyst has a diameter of 1.5 to 8.0 mm, preferably 3.2 to 5.8 mm, preferably 3.3 to 5.7 mm, preferably 3.4 to 5.5 mm.

In a preferred embodiment of the present invention the centre of the catalyst has a diameter of 1.0 to 7.7 mm, preferably 2.2 to 7.2 mm, preferably 3.0 to 4.35 mm, preferably 3.7 to 4.3 mm, preferably 3.9 to 4.1 mm, in particular 4.0 mm.

In a preferred embodiment of the present invention the ratio of the radius of the centre to the distance between the geometrical surface of the catalyst and the circumference of the catalyst centre is from 15 to 2, preferably 12 to 4, preferably 10 to 6, particularly 9 to 7 and most preferably 8.

In a preferred embodiment of the present invention the, preferably spherical, supported Pd-catalyst has a diameter of 1.5 to 3.0 mm, which catalyst comprises an outer shell region having a maximum depth of 250 $\mu$m, 220 $\mu$m, 200 $\mu$m or 150 $\mu$m and a centre, wherein at least 90 % of the total amount of the at least one metal selected from the group consisting of palladium and the at least two metals selected from the group consisting of platinum, silver, manganese and bismuth are distributed in the outer shell region. The centre of said preferred catalyst has a diameter of 1.0 to 2.7 mm and the ratio of the radius of the centre to the distance between the geometrical surface of the catalyst and the circumference of the catalyst centre is from 2 to 15.

In a preferred embodiment of the present invention the supported Pd-catalyst has a diameter of 3.0 to 8.0 mm. The centre of said preferred catalyst has a diameter of 2.2 to 7.7 mm and the ratio of the radius of the centre to the distance between the geometrical surface of the catalyst and the circumference of the catalyst centre is from 2 to 15.

The problem of the present invention is also solved by a method for preparing a Pd-catalyst comprising combining palladium with at least two metals selected from the group consisting of platinum, silver, manganese and bismuth.

The problem of the present invention is also solved by a method for preparing a supported Pd-catalyst comprising combining palladium and at least two metals selected from the group consisting of platinum, silver, manganese and bismuth, with a support.

The problem of the present invention is also solved by a method for preparing a supported Pd-catalyst comprising combining palladium with a support comprising at least two metals selected from the group consisting of platinum, silver, manganese and bismuth.

The problem of the present invention is also solved by a method for preparing a supported Pd-catalyst comprising combining at least two metals selected from the group consisting of platinum, silver, manganese and bismuth with a support comprising palladium.

In a preferred embodiment of the present invention the method for producing the supported Pd-catalyst comprises the following steps:

(a) providing a support comprising at least two metals selected from the group consisting of platinum, silver, manganese and bismuth,
(b) impregnating the support of step (a) with a liquid medium comprising palladium,
(c) drying the impregnated support of step (b) and
(d) treating the dried, impregnated support obtained in step (c) so as to obtain a supported Pd-catalyst.

In a preferred embodiment of the present invention the method for producing the supported Pd-catalyst comprises the following steps:

(i) providing a support,
(ii) impregnating the support of step (i) with a liquid medium comprising at least two metals selected from the group consisting of platinum, silver, manganese and bismuth,

(iii) drying the impregnated support of step (ii),
(iv) impregnating the dried, impregnated support of step
(iii) with a liquid medium comprising palladium,
(v) drying the impregnated support of step (iv) and
(vi) treating the dried, impregnated support obtained in step (v) so as to obtain a supported Pd-catalyst.

In a preferred embodiment the present invention foresees a method for producing a monolithic or honeycomb-based Pd catalyst comprising applying palladium and at least two metals selected from the group consisting of platinum, silver, manganese and bismuth to the monolithic or honeycomb body.

In a preferred embodiment of the present invention the method for producing the monolithic or honeycomb-based Pd catalyst comprises the following steps:

(s) providing a monolithic or honeycomb body,
(t) coating the monolithic or honeycomb body provided in step (s) with a catalyst support,
(u) impregnating the coated monolithic or honeycomb body obtained in step (t) with a liquid medium comprising at least two metals selected from the group consisting of platinum, silver, manganese and bismuth,
(w) drying the impregnated, coated monolithic or honeycomb body obtained in step (u),
(x) impregnating the dried monolithic or honeycomb body obtained in step (w) with a liquid medium comprising palladium,
(y) drying the impregnated monolithic or honeycomb body obtained in step (x) and
(z) treating the impregnated and dried monolithic or honeycomb body obtained in step (y) so as to obtain the monolithic or honeycomb-based Pd-catalyst.

In a preferred embodiment of the present invention the impregnation and drying steps are repeated, until the desired contents of the different metals are present in the supported Pd-catalyst.

In a preferred embodiment of the present invention the impregnation steps (ii) and (iv) are carried out simultaneously, thus rendering the intermediate drying step (iii) unnecessary.

In a preferred embodiment of the present invention the impregnation steps (u) and (x) are carried out simultaneously, thus rendering the intermediate drying step (w) unnecessary.

In a preferred embodiment of the present invention the impregnation step (iv) and the subsequent drying step (v) are carried out before the impregnation step (ii).

In a preferred embodiment of the present invention the impregnation step (x) and the subsequent drying step (y) are carried out before the impregnation step (u).

In a preferred embodiment of the present invention the metals in the liquid medium of step (b), (u), (x), (ii) and/or (iv) are present in form of their salts, preferably in form of their nitrates.

In a preferred embodiment of the present invention the impregnation in step (b), (u), (x), (ii) and/or (iv) is carried out by spraying the liquid medium onto the support. By spraying the liquid mediums onto the catalyst support a particular homogeneous distribution is obtained.

In a preferred embodiment of the present invention the spraying of the liquid medium onto the catalyst is carried out with a nozzle capable of finely dispersing liquids such as solutions or suspensions.

In a preferred embodiment of the present invention the liquid medium is homogeneously distributed in the pores of the catalyst support.

In a preferred embodiment of the present invention the drying temperature in step (c), (w), (y), (iii) and/or (v) is from 80 to 350 °C, preferably 90 to 300 °C, preferably 100 to 250 °C, preferably 110 to 200 °C and in particular 120 to 150 °C.

In a preferred embodiment of the present invention the drying of the impregnated precursor in step (c), (w), (y), (iii) and/or (v) is carried out to a dry residue of 99 %, preferably 99.5 %, preferably 99.9 % and in particular 100 %.

In a preferred embodiment the drying step is performed by microwave treatment.

In a preferred embodiment of the present invention the drying can be carried out under static or dynamic conditions, for instance in a fixed bed, or in a moving bed.

In a preferred embodiment of the present invention the catalyst according to the present invention is inerted, preferably at a temperature from 300 to 350°C, preferably 350°C, preferably after the drying and preferably before the treating, preferably the calcining.

In a preferred embodiment of the present invention the inerting is carried out at the same temperature as the drying.

In a preferred embodiment of the present invention the dried impregnated precursor obtained in step (c), (y) and/or (v) is in a further step (c''), (y'') and/or (v'') flushed with hydrogen, preferably before the treating, in particular the calcining, in step (d), (z) and/or (vi).

In a preferred embodiment of the present invention the flushing with hydrogen carried out in step (c''), (y") and/or (v") is performed for 2 to 20 minutes, preferably 2 to 5 min.

In a preferred embodiment the present invention comprises both the inerting and the flushing step.

A preferred embodiment foresees that subsequently to the drying step (c), (y) and/or (v) the dried, impregnated catalyst is inerted, subsequently flushed with hydrogen and then treated, preferably calcined, in step (d), (z) and/or (vi).

In a preferred embodiment of the present invention the treating, especially the calcining, in step (d), (z) and/or (vi) is carried out at a temperature from 200 to 700 °C, preferably 300 to 700 °C and in particular 400 to 700 °C.

In a preferred embodiment of the present invention the process foresees to subject the catalyst to a nitrogen stream while the temperature is increased to the temperature of treating, preferably calcining.

In a preferred embodiment of the present invention the treatment, preferably calcining, is carried out in a fixed bed or in a moving bed reactor.

In a preferred embodiment of the present invention the treating in step (d), (z) and/or (vi) is carried out with a nitrogen-containing gas, preferably air or nitrogen.

In a preferred embodiment of the present invention the selectivity and/or durability of the catalysts is further increased by adding before and/or during the treating in step (d), (z) and/or (vi) to the nitrogen-containing gas, preferably to the nitrogen, used therefore a gas which is preferably selected from the group consisting of hydrogen, air, oxygen, water vapour and $NO_x$, with a content from 0 to 500 ppmv (parts per million per volume), preferably 10 to 400 ppmv, preferably 20 to 300 ppmv, preferably 30 to 200 ppmv and in particular 50 to 100 ppmv.

In a preferred embodiment of the present invention the Pd-catalyst is reduced after the treating in step (d), (z) and/or (vi) in a step (e), (z') and/or (vii), to be preferably suitable for the hydrogenation of a hydrocarbon feed, preferably at a temperature of 50 °C to 650 °C, preferably 150 to 500 °C and in particular 400 °C for four hours in a hydrogen stream.

In a preferred embodiment of the present invention the Pd-catalyst is dried, preferably at a temperature of 120 °C, before said Pd-catalyst is reduced.

In a preferred embodiment of the present invention during and/or after the reduction of the Pd-catalyst the presence of oxygen, oxygen containing gases and/or gases causing oxidation of the catalytically active metals is prevented, due to the fact that reduced metals, preferably with the oxidation state of zero, can be oxidized easily to their oxides.

In a preferred embodiment of the present invention the dried and impregnated support is treated, preferably is calcined, in step (d), (z) and/or (vi) for 1 to 20 hours, preferably 2 to 18 hours, preferably 3 to 15 hours, preferably 4 to 10 hours and in particular 4 to 8 hours.

In a preferred embodiment of the present invention the dried and impregnated support is treated, preferably is calcined, in step (d), (z) and/or (vi) at a GHSV (gas hourly space velocity) from 10 to 5000 v/vh, preferably 1000 to 4500 v/vh and in particular 3000 to 4000 v/vh.

In a preferred embodiment of the present invention the catalyst is cooled after the treating, preferably calcining, in step (d), (z) and/or (vi) in a nitrogen containing gas, for instance under nitrogen protective atmosphere, preferably to a temperature below 50 °C.

The supported Pd-catalysts can be used preferably for the hydrogenation, preferably for the selective hydrogenation, preferably for the selective hydrogenation of the hydrocarbon feed, preferably comprising a first and a second group of unsaturated hydrocarbons, wherein preferably the first group is hydrogenated thereby, with a particular long catalyst lifetime allowing significantly increased cycle times. Based on the particular high durability of said catalyst hydrogenation processes can be repeated more often, before the said catalyst has to be regenerated. Advantageously, the catalyst according to the present invention reduces the formation of green oil.

The technical problem of the present invention is also solved by a method for the hydrogenation of a hydrocarbon feed, preferably in gaseous phase, in particular a method for the selective hydrogenation of a hydrocarbon feed containing unsaturated hydrocarbons, in particular a first group of undesired, that means highly unsaturated hydrocarbons, in particular aromatics, alkynes and/or di, tri- or polyunsaturated hydrocarbons, particularly alkadienes, alkatrienes or alkapolyenes, such as acetylene, propyne, propadiene, butadienes, vinylacetylene, butynes, phenylacetylene and/or styrene and a second group of desired, that means less unsaturated hydrocarbons, in particular monounsaturated hydrocarbons, namely alkenes, particularly ethylene, wherein the hydrocarbon feed is contacted under suitable hydrogenating conditions with the catalyst according to the present invention so as to remove the undesired first group of unsaturated hydrocarbons, in particular to hydrogenate them, preferably to a desired less unsaturated hydrocarbon, thereby leaving the second group of unsaturated hydrocarbons in their monounsaturated form.

Accordingly, the present invention also relates to a method for the hydrogenation, preferably for the selective hydrogenation, of a hydrocarbon feed, preferably comprising a first and a second group of unsaturated hydrocarbons, wherein the hydrocarbon feed is contacted under suitable hydrogenating conditions with the catalyst of the present invention and the unsaturated hydrocarbons in the hydrocarbon feed, preferably in the first group, are hydrogenated. Thus, the use of a catalyst according to the present invention enables a process for the selective hydrogenation of highly unsaturated hydrocarbons in the presence of less unsaturated hydrocarbons.

The catalyst according to the present invention can be used in a hydrogenation process, in particular a selective hydrogenation of a hydrogen feed comprising unsaturated hydrocarbons, with a particular long catalyst lifetime allowing significantly increased cycle times. Based on the particular high durability of said catalyst hydrogenation processes can be repeated more often, preferably in case of a batch-wise operation, before the said catalyst has to be regenerated or in a continuous fixed bed the lifetime and/or conversion is increased. Advantageously, the catalyst according to the present invention reduces the formation of higher hydrocarbons.

Thus, in a preferred embodiment of the present invention the hydrogenation is carried out in the gas phase.

In a further preferred embodiment, the present hydrogenation process is carried out according to the conditions of a front-end or tail-end hydrogenation process, preferably for the hydrogenation of C2 to C4 hydrocarbons.

In a preferred embodiment of the hydrogenation method of the present invention the hydrocarbon feed comprises acetylene in its first group of unsaturated hydrocarbons, in particular in the presence of less unsaturated hydrocarbons. In a preferred embodiment, the present process foresees to preferably reduce acetylene to ethylene, in particular in the presence of less unsaturated hydrocarbons, preferably ethylene.

In a preferred embodiment acetylene is hydrogenated selectively.

In a preferred embodiment of the present invention the hydrocarbon feed is contacted in the hydrogenation process with the catalyst according to the present invention at a temperature from 10 to 250 °C, preferably 30 to 200 °C, preferably 40 to 180 °C and in particular 40 to 100 °C.

In a preferred embodiment of the present invention the hydrocarbon feed is contacted in the hydrogenation process with the catalyst at a pressure from 0.5 to 90 bars, preferably 0.5 to 60 bars, preferably 5 to 20 bars, preferably 0.5 to 2 bars and in particular 10 to 20 bars.

In a preferred embodiment of the present invention the hydrocarbon feed is conducted in the hydrogenation process with the catalyst at a GHSV (gas hourly space velocity) from 1000 to 15000 v/vh, from 5000 to 12000 v/vh, preferably from 3000 to 7000 v/vh and in particular 5000 to 7000 v/vh.

In a preferred embodiment of the present invention the hydrocarbon feed is contacted in the hydrogenation process with a catalyst without the use of carbon monoxide as moderator. Furthermore, the hydrogenation can be carried out without carbon monoxide, namely in a carbon monoxide-free process.

In a preferred embodiment of the present invention the molar ratio of hydrogen to the acetylene is from 0.8 to 1.8, preferably 1.0 to 1.5 and in particular 1.0 to 1.3, particularly 1.2.

In a preferred embodiment of the present invention the molar ratio of the hydrogen to the acetylene is from 1.8 to 100, preferably 1.8 to 70, preferably 1.8 to 30 and in particular 1.8 to 10.

In a preferred embodiment of the present invention poisoning metals, such as mercury and arsenic, are removed prior to the hydrogenation process, preferably using a guard bed.

In a preferred embodiment for the process of hydrogenation the hydrocarbon feed is contacted with hydrogen to obtain the hydrogenated products.

The invention is further illustrated by the following Examples.

Examples

Production of the catalysts

Catalyst A (Pd; comparison)

An $\alpha$-Al$_2$O$_3$ carrier was impregnated dropwise with an aqueous Pd(NO$_3$)$_2$ solution to yield a Pd content of 0.54 wt.%. The impregnated carrier was dried in a microwave oven at 300 W and finally calcined in air at 350°C for 3.5 hours.

Catalyst B (Pd+Ag; comparison)

An $\alpha$-Al$_2$O$_3$ carrier was impregnated dropwise with an aqueous Pd(NO$_3$)$_2$ solution to yield a Pd content of 0.53 wt.%. The impregnated carrier was dried in a microwave oven at 300 W. After drying, it was impregnated dropwise with an aqueous AgNO$_3$ solution to yield a Ag content of 0.8 wt.%. The impregnated carrier was dried in a microwave oven at 300 W and finally calcined in air at 350°C for 3.5 hours.

Catalyst C (Pd+Pt+Bi; according to the invention)

An $\alpha$-Al$_2$O$_3$ carrier was impregnated dropwise with an aqueous Pt(NO$_3$)$_2$ and Bi(NO$_3$)$_2$ solution to yield a Pt content of 0.2 wt.% and a Bi content of 0.2 wt.%. The impregnated carrier was dried in a microwave oven at 300 W. After drying, it was impregnated dropwise with an aqueous Pd(NO$_3$)$_2$ solution to yield a Pd content of 0.54 wt.%. The impregnated carrier was dried in a microwave oven at 300 W and finally calcined in air at 350°C for 3.5 hours.

Catalyst D (Pd+Ag+Mn; according to the invention)

An α-Al$_2$O$_3$ carrier was impregnated dropwise with an aqueous AgNO$_3$ and Mn(NO$_3$)$_2$ solution to yield a Ag content of 0.8 wt.% and a Mn content of 0.2 wt.%. The impregnated carrier was dried in a microwave oven at 300 W. After drying, it was impregnated dropwise with an aqueous Pd(NO$_3$)$_2$ solution to yield a Pd content of 0.53 wt.%. The impregnated carrier was dried in a microwave oven at 300 W and finally calcined in air at 350°C for 3.5 hours.

Catalytic properties of Catalysts A-D

The catalytic properties of Catalysts A-D were characterized by the following test.
- GHSV 7,000 v/vh
-

| feed composition (vol.%) | 1.0 C$_2$H$_2$ | 1.0 H$_2$ |
| --- | --- | --- |
| | 30 C$_2$H$_4$ | 1.0 C$_3$H$_8$ |
| | rest : Ar | |

- pressure: 10 bar
- temperature: 40-60 °C (adjusted to achieve 60% conversion of acetylene)
Prior to testing, the catalysts were dried at 120 °C and reduced in hydrogen at 400 °C for 4 hours.
In the catalytic tests, the selectivity towards ethylene at 60% conversion of acetylene was determined, the results of which are shown in Table 1. The acetylene conversion and selectivity towards ethylene are defined as follows.
The acetylene conversion is defined as:

$$X_{Ac} = \frac{\dot{n}^0_{Ac} - \dot{n}_{Ac}}{\dot{n}^0_{Ac}} \cdot 100\%$$

The selectivity towards ethylene is defined as:

$$S_{Ey} = \frac{(\dot{n}^0_{Ac} - \dot{n}_{Ac}) - (\dot{n}_{Ea} - \dot{n}^0_{Ea}) - 2(\dot{n}_{C_4} - \dot{n}^0_{C_4})}{(\dot{n}^0_{Ac} - \dot{n}_{Ac})} \cdot 100\%$$

n = mole flow
n° = mole flow at the inlet
Ac = acetylene
Ey = ethylene
Ea = ethane

Table 1

| Catalyst | Selectivity towards ethylene (%) |
| --- | --- |
| A (comparison) | 36.3 |
| B (comparison) | 61.3 |
| C (invention) | 70.7 |
| D (invention) | 75.2 |

From Table 1 it is evident that Catalysts C and D according to the present invention advantageously provide a substantially higher degree of selectivity towards ethylene than comparison Catalysts A and B.

**Claims**

1.  A Pd-catalyst, comprising palladium and at least two metals selected from the group consisting of platinum, silver, manganese and bismuth.

2.  The Pd-catalyst according to claim 1, which comprises a support.

3.  The Pd-catalyst according to claim 1 or 2, which comprises a monolithic or honeycomb body.

4.  The Pd-catalyst according to claim 2, wherein the support is $Al_2O_3$ or a modification thereof.

5.  The Pd-catalyst according to any one of the preceding claims, wherein the at least two metals comprise a first metal which is platinum or silver and a second metal which is manganese or bismuth.

6.  The Pd-catalyst according to claim 5, wherein the first metal is platinum and the second metal is bismuth.

7.  The Pd-catalyst according to claim 5, wherein the first metal is silver and the second metal is manganese.

8.  The Pd-catalyst according to any one of claims 5-7, wherein the weight ratio of palladium to the total of the first and second metals is from 2:1 to 1:4.

9.  The Pd-catalyst according to any one of claims 5-7, wherein the weight ratio of palladium to the first metal is from 3:1 to 1:3.

10. The Pd-catalyst according to any one of claims 5-7, wherein the weight ratio of palladium to the second metal is from 5:1 to 1:1.

11. A method for preparing a Pd-catalyst comprising combining palladium with at least two metals selected from the group consisting of platinum, silver, manganese and bismuth.

12. A method for the hydrogenation, preferably for the selective hydrogenation, of a hydrocarbon feed, preferably comprising a first and a second group of unsaturated hydrocarbons, wherein the hydrocarbon feed is contacted under suitable hydrogenating conditions with the catalyst according to any one of claims 1 to 10 and the unsaturated hydrocarbons in the hydrocarbon feed, preferably in the first group, are hydrogenated.

13. The method for the hydrogenation according to claim 12, wherein the hydrocarbon feed comprises acetylene in its first group of unsaturated hydrocarbons.

Europäisches
Patentamt

European
Patent Office

Office européen
des brevets

**EUROPEAN SEARCH REPORT**

Application Number

EP 11 18 1793

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 947 236 A1 (ENGELHARD CORP [US]) 6 October 1999 (1999-10-06) * paragraph [0051] - paragraph [0054] * | 1-11 | INV. C10G45/40 B01J23/50 C07C5/08 C07C7/167 |
| X | US 5 516 851 A (FLICK KLEMENS [DE] ET AL) 14 May 1996 (1996-05-14) * column 4, line 54 - line 63; examples 4,5 * | 12,13 | |
| A | US 2002/068843 A1 (DAI WEI [CN] ET AL) 6 June 2002 (2002-06-06) * paragraph [0009] * | 1-13 | |
| X | WO 03/033136 A2 (CATALYTIC DISTILLATION TECH [US]) 24 April 2003 (2003-04-24) * page 7, paragraph 2; claims 1-4,13 * | 1-13 | |
| X | US 6 350 717 B1 (FRENZEL ANDREA [DE] ET AL) 26 February 2002 (2002-02-26) * column 3, line 51 - line 56 * * column 5, line 22 - line 24; claims 1,3-5 * | 1-13 | |

TECHNICAL FIELDS
SEARCHED (IPC)

C10G
B01J
C07C

~~The present search report has been drawn up for all claims~~

| Place of search | Date of completion of the search | Examiner |
|---|---|---|
| Munich | 28 February 2012 | Deurinck, Patricia |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

| | |
|---|---|
| Europäisches Patentamt | **Application Number** |
| European Patent Office | EP 11 18 1793 |
| Office européen des brevets | |

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-13(partially)

☐ The present supplementary European search report has been drawn up for those parts of the European patent application which relate to the invention first mentioned in the claims (Rule 164 (1) EPC).

13

Europäisches
Patentamt

European
Patent Office

Office européen
des brevets

**LACK OF UNITY OF INVENTION
SHEET B**

Application Number

EP 11 18 1793

The Search Division considers that the present European patent application does not comply with the requirements of unity of invention and relates to several inventions or groups of inventions, namely:

1. claims: 1-13(partially)

   A Pd-catalyst comprising palladium, platinum and silver, its preparation and its use in a selective hydrogenation process.
   ---

2. claims: 1-13(partially)

   A Pd-catalyst comprising palladium, platinum and manganese, its preparation and its use in a selective hydrogenation process.
   ---

3. claims: 1-13(partially)

   A Pd-catalyst comprising palladium, platinum and bismuth, its preparation and its use in a selective hydrogenation process.
   ---

4. claims: 1-13(partially)

   A Pd-catalyst comprising palladium, silver and manganese, its preparation and its use in a selective hydrogenation process.
   ---

5. claims: 1-13(partially)

   A Pd-catalyst comprising palladium, silver and bismuth, its preparation and its use in a selective hydrogenation process.
   ---

6. claims: 1-13(partially)

   A Pd-catalyst comprising palladium, manganese and bismuth, its preparation and its use in a selective hydrogenation process.
   ---

7. claims: 1-13(partially)

   A Pd-catalyst comprising palladium, platinum, silver and manganese, its preparation and its use in a selective hydrogenation process.
   ---

8. claims: 1-13(partially)

Europäisches
Patentamt

European
Patent Office

Office européen
des brevets

**LACK OF UNITY OF INVENTION
SHEET B**

Application Number

EP 11 18 1793

The Search Division considers that the present European patent application does not comply with the
requirements of unity of invention and relates to several inventions or groups of inventions, namely:

> A Pd-catalyst comprising palladium, platinum, silver and bismuth, its preparation and its use in a selective hydrogenation process.
> ---

9. claims: 1-13(partially)

> A Pd-catalyst comprising palladium, platinum, manganese and bismuth, its preparation and its use in a selective hydrogenation process.
> ---

10. claims: 1-13(partially)

> A Pd-catalyst comprising palladium, silver, manganese and bismuth, its preparation and its use in a selective hydrogenation process.
> ---

11. claims: 1-13(partially)

> A Pd-catalyst comprising palladium, platinum, silver, manganese and bismuth, its preparation and its use in a selective hydrogenation process.
> ---

**ANNEX TO THE EUROPEAN SEARCH REPORT
ON EUROPEAN PATENT APPLICATION NO.**

EP 11 18 1793

This annex lists the patent family members relating to the patent documents cited in the above-mentioned European search report.
The members are as contained in the European Patent Office EDP file on
The European Patent Office is in no way liable for these particulars which are merely given for the purpose of information.

28-02-2012

| Patent document cited in search report | | | Publication date | Patent family member(s) | | | Publication date |
|---|---|---|---|---|---|---|---|
| EP | 0947236 | A1 | 06-10-1999 | EP | 0947236 | A1 | 06-10-1999 |
| | | | | US | 6074973 | A | 13-06-2000 |
| US | 5516851 | A | 14-05-1996 | CA | 2135733 | A1 | 17-05-1995 |
| | | | | CN | 1111251 | A | 08-11-1995 |
| | | | | DE | 4339138 | A1 | 18-05-1995 |
| | | | | EP | 0653243 | A1 | 17-05-1995 |
| | | | | ES | 2154660 | T3 | 16-04-2001 |
| | | | | US | 5516851 | A | 14-05-1996 |
| US | 2002068843 | A1 | 06-06-2002 | CN | 1466486 | A | 07-01-2004 |
| | | | | JP | 4339681 | B2 | 07-10-2009 |
| | | | | JP | 2004520932 | A | 15-07-2004 |
| | | | | RU | 2278731 | C2 | 27-06-2006 |
| | | | | US | 2002068843 | A1 | 06-06-2002 |
| | | | | WO | 03015916 | A1 | 27-02-2003 |
| WO | 03033136 | A2 | 24-04-2003 | AR | 036628 | A1 | 22-09-2004 |
| | | | | AU | 2002341704 | A1 | 28-04-2003 |
| | | | | CN | 1604955 | A | 06-04-2005 |
| | | | | TW | 583168 | B | 11-04-2004 |
| | | | | US | 2005010070 | A1 | 13-01-2005 |
| | | | | US | 2005203320 | A1 | 15-09-2005 |
| | | | | WO | 03033136 | A2 | 24-04-2003 |
| US | 6350717 | B1 | 26-02-2002 | CA | 2281247 | A1 | 04-03-2000 |
| | | | | CN | 1253853 | A | 24-05-2000 |
| | | | | DE | 19840373 | A1 | 09-03-2000 |
| | | | | EP | 0985447 | A2 | 15-03-2000 |
| | | | | JP | 2000153154 | A | 06-06-2000 |
| | | | | KR | 20000022888 | A | 25-04-2000 |
| | | | | TW | 442329 | B | 23-06-2001 |
| | | | | US | 6350717 | B1 | 26-02-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 2603578
- US 2802889 A
- DE 19757990
- US 4484015 A
- DE 19840373
- KR 20000059743
- US 7453017 B
- US 6255548 B
- WO 2011107565 A